(19) Europäisches Patentamt
European Patent Office
Office européen des brevets

(11) Publication number: 0 227 340 B1

(12) # EUROPEAN PATENT SPECIFICATION

(45) Date of publication of patent specification: **15.04.92**

(51) Int. Cl.5: **C09D 183/04**, A61M 5/00

(21) Application number: **86309384.5**

(22) Date of filing: **02.12.86**

(54) **Film-forming composition.**

(30) Priority: **03.12.85 US 804209**

(43) Date of publication of application:
**01.07.87 Bulletin 87/27**

(45) Publication of the grant of the patent:
**15.04.92 Bulletin 92/16**

(84) Designated Contracting States:
**BE CH DE ES FR GB IT LI NL SE**

(56) References cited:
**EP-A- 0 175 355**
**US-A- 4 472 470**

(73) Proprietor: **Deseret Medical, Inc.**
**9450 South State Street**
**Sandy Utah 84070(US)**

(72) Inventor: **Spielvogel, David E.**
**45 Graham Drive**
**Springboro, Ohio 45066(US)**
Inventor: **Zdrahala, Richard J.**
**114 Williamsburg Lane**
**Dayton, Ohio 45459(US)**

(74) Representative: **Jones, Michael Raymond et al**
**HASELTINE LAKE & CO. Hazlitt House 28**
**Southampton Buildings Chancery Lane**
**London WC2A 1AT(GB)**

EP 0 227 340 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The instant invention relates to a film-forming silicone composition having a non-reactive lubricating component dispersed or distributed within a reactive component such that when the composition is applied to or used in conjunction with a substrate surface, it coats and adheres to the surface while providing surface lubrication.

Certain silicone coating compounds are well known in the art for their lubricating properties. Those compounds disclosed have various disadvantages and shortcomings which the inventive compositions seek to overcome. U.S. Patent 3,574,073 discloses the use of cured organosiloxane copolymers on fine cutting edges such as razor blades and hypodermic needles. The copolymers of this reference consist of:

(1) 5 to 20 weight percent of polymeric units of the formula:

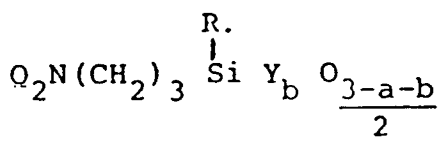

Wherein R is an alkyl radical $C_{1-6}$; Y is -OH and OR' where R' is an alkyl radical up to 3 carbons; Q is $-CH_3$ or $-CH_2CH_2NH_2$; a and b have a value of 0 or 1 where their sum is from 0 to 2; and

(2) 80 to 95 weight percent of polymeric units of the formula

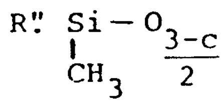

wherein R'' is -OH or $-CH_3$ radicals and c has a value of 1 or 2.

The coatings of this reference suffer several shortcomings and disadvantages. To begin with, these polymers are moisture cured. Although some lubricating effect is obtained while these films are in the uncured state, it takes from two to ten days to obtain a fully cured coating. The polymers of this reference are amino terminated, thus the surfaces of this polymer due to the amine functionality are alkaline in nature. This alkalininity may potentially initiate a hemolytic and/or thrombogenic reaction when used in articles which contact blood.

Another known silicone lubricant widely used in the biomedical field on hypodermic needles is polydimethylsiloxane (PDMS). While the medical grade fluids have the advantage of being chemically inert, these materials have a tendency to creep or migrate from the surface to which they are applied. For example, in the case of a hypodermic needle coated with PDMS, the coating might be substantially removed due to frictional forces during penetration of the skin and vein, making subsequent removal of the needle difficult and painful to the patient. Migration during storage and inadvertant removal during processing is also a concern.

Heretofore, the prior art has not disclosed a lubricating siloxane composition which cures quickly to an adherent film, without migration problems or long cure times. It is apparent that a need exists for a lubricating composition which when applied to substrate surfaces such as hypodermic needles, cutting edges, razor blades and the like, adheres to the substrate surface and provides lubrication, durability and biocompatibility. In the case of hypodermic needles, the lubricating composition serves to decrease the penetration force into the skin or vein, as well as decrease the drag and retract force during removal.

According to the present invention, there is provided a film-forming composition comprising:

(a) a reactive component for providing adhesion, said reactive component comprising:

(1) a siloxane polymer having a formula selected from:

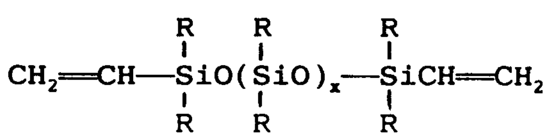

(I)

and

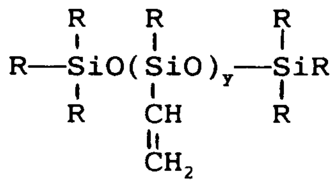

(II)

and

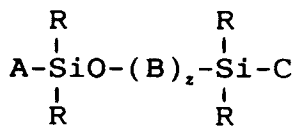

(III)

wherein R is selected from $C_{1-20}$ alkyl, haloalkyl, aryl, haloaryl, cycloalkyl, silacyclopentyl, aralkyl and mixtures thereof; x is from 60 to 1000; y is from 3 to 25; z is 3 to 25 to 60 to 1000; $(B)_z$ is a mixture or combination, in any order, of

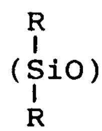

and

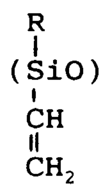

units such that the total number of units is equal to z, each of A and C is independently vinyl or R, wherein R is as defined above;

(2) a siloxane cross-linking polymer having the formula:

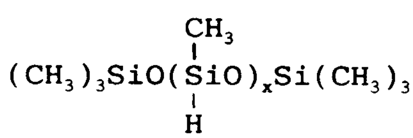

(IV)

wherein x is from 8 to 12; and

(3) a siloxane chain-extending polymer having the formula:

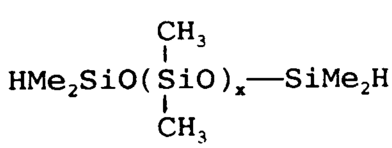

(V)

wherein x is from 140 to 160; and

(b) a non-reactive lubricating siloxane polymer component having the formula:

$$(R)_3SiO(\underset{R}{\overset{R}{|}}\!\!\!\!SiO)_xSi(R)_3 \qquad (VI)$$

wherein R is selected from $C_{1-20}$ alkyl, haloalkyl, aryl, haloaryl, cycloalkyl, silacyclopentyl, aralkyl, and mixtures thereof; and x is from 20 to 1,350;

wherein the mole ratio of vinyl groups to hydrogen atoms is within the range of from 0.010:1 to 0.20:1.

Preferably, the composition is one wherein the siloxane polymer (1) of the reactive component (a) is present in an amount in the range from 3% to 35% by weight of the total composition; and/or wherein the siloxane cross-linking polymer (2) of the reactive component (a) is present in an amount in the range from 0.3% to 5.5% by weight of the total composition; and/or wherein the siloxane chain-extending polymer (3) of the reactive component (a) is present in an amount in the range from 2.5% to 50.5% by weight of the total composition.

The molar ratio of vinyl groups to pendant and terminal hydrogen atoms is in the range from 0.010:1 to 0.20:1.

The preferred molar ratio of pendant hydrogen atoms of the cross-linking polymer (2) to the terminal hydrogen atoms of the chain-extending polymer (3) is in the range from 5.0:1 to 20:1.

Preferably the composition is one wherein the viscosity of the reactive component (a) is in the range from 100 to 100,000 centistokes; and/or wherein the average molecular weight per cross-link of the total reactive component (a) is in the range from 5,000 to 75,000.

Preferably, the siloxane polymer (1) of the reactive component (a) is a mixture of two or more of the polymers of formulae I to III.

Preferably the composition is one wherein the non-reactive lubricating component (b) is present in an amount in the range from 10% to 90% by weight of the total composition; and/or wherein the viscosity of the non-reactive siloxane polymer (b) is in the range from 20 to 300,000 centistokes; and/or wherein the average molecular weight of the non-reactive siloxane polymer (b) is in the range from 1900 to 100,000.

Preferably there is additionally incorporated in the composition a reaction catalyst selected from platinum, palladium, rhodium and mixtures thereof.

Preferably, the siloxane polymer (1) having the formula I of the reactive component (a) is one having the following formula:

$$CH_2{=}CH\text{-}\underset{CH_3}{\overset{CH_3}{Si}}O(\underset{CH_3}{\overset{CH_3}{Si}}O)_x\underset{CH_3}{\overset{CH_3}{Si}}CH{=}CH_2$$

wherein x is in the range from 200 to 500.

Preferably, the non-reactive lubricating component (b) comprises one or more compounds of the formula

$$(CH_3)_3SiO(\underset{CH_3}{\overset{CH_3}{Si}}O)_zSi(CH_3)_3 \qquad (VII)$$

wherein z is in the range from 70 to 1,350;

The preferred respective molecular weight ranges of the siloxane polymers (1), (2) and (3) are from 5000 to 75,000, 400 to 7,500 and 400 to 37,000.

In a film formed from the composition, the reactive component (a) is a chemically crosslinked, surface adherent polysiloxane, which serves as a matrix for the non-reactive component (b) dispersed therein. Each of the three siloxane polymers (1) to (3) of the reactive component (a) is required to achieve the durability and adherent properties required for the intended usefulness of the compositions as a film or coating. Preferably a mixture of polymers is used for each of the three types of required polymers in the reactive

component (a). For example, a mixture of siloxane polymers of the formula I to III can be used as the siloxane polymer (1); a mixture of polymers of the formula IV can be used as the siloxane polymer (2); and a mixture of chain-extending polymers of the formula V can be used as the siloxane polymer (3).

The siloxane polymer (1) of the reactive component (a) is preferably present in an amount in the range from 3% to 35% by weight of the total film-forming composition, as indicated above; it is more preferably in an amount of from 10% to 30% weight percent. This polymer corresponds to the structural formulae I, II or III given above wherein each of x, y and z are in the range mentioned above, and preferably x is in the range from 200 to 320. The value z is preferably 3 to 25 or 60 to 1000.

It is preferred that a mixture of siloxane polymers selected from the formulae I, II, III be used as the siloxane polymer (1) of the reactive component (a). Most preferably this mixture comprises a mixture of two different molecular weight siloxane polymers of the formula VII, wherein one of the polymers has an average molecular weight in the range from 5,000 to 25,000 and preferably about 16,000, and the other polymer has an average molecular weight in the range from 30,000 to 75,000 and preferably about 38,000. The lower molecular weight siloxane polymer is preferably present in an amount in the range from 20% to 80%, and more preferably about 60% by weight of this mixture; and the higher molecular weight siloxane polymer is preferably present in an amount in the range from 80% to 20%, and more preferably about 40% by weight of this mixture.

The siloxane polymer (2) of the reactive component (a) is preferably present in an amount in the range mentioned above, namely from 0.3 to 5.5% by weight of the total composition and more preferably in an amount in the range from 0.5 to 4.0%, and has the formula IV wherein x is in the range from 8 to 12 and preferably about 10.

Preferably the molecular weight range of the siloxane polymer (2) is from 400 to 7,500.

The final requirement of the reactive component (a) is a siloxane chain-extending polymer (3) having the formula V above. These compounds are preferably present in the reactive component (a) in an amount in the range from 2.5% to 50 or 50.5%, and more preferably in the range from 5% to 40% by weight of the reactive component (a). In formula V, x is in the range from 140 to 170 and preferably in the range from 150 to 160, or 140 to 160.

Preferably a mixture of these polymers is also used comprising two different molecular weight materials. For example, a preferred embodiment incorporates a range from 2% to 5% by weight of the mixture of the siloxane polymer (2) having an average molecular weight in the range from 400 to 7,500, and preferably about 1900, in admixture with a range from 98% to 95% of a siloxane polymer (3) having an average molecular weight of in the range from 400 to 37,000 and preferably about 12,000.

The three required siloxane polymers (1) to (3) of the reactive component (a) are preferably present in relative weight ratios of in the range from 0.2:5:1 to 2:20:1 and preferably in the range from 0.4:5:1 to 1.5:16:1. In these ratios, an adherent, fast curing film is obtained with the added advantage that it is optically clear and free of cloudiness or opaque appearance.

The non-reactive component (b) of the compositions of the present invention is responsible for the lubricating properties of the resultant films and coatings. This component comprises a siloxane polymer of formula VI, preferably having an average molecular weight in the range from 1900 to 100,000, more preferably from 5,000 to 100,000. Generally this corresponds to a viscosity in the range from 20 to about 300,000 centistokes (cstks) mentioned above. The non-reactive component is preferably present in an amount in the range from 10% to 90%, and more preferably in the range from 70% to 80% by weight of the total composition.

Preferably, the non-reactive component (b) has the following formula IX:

$$(CH_3)_3Si(\overset{\displaystyle CH_3}{\underset{\displaystyle CH_3}{|\!\!\!\!\!\;\;Si}}O)_wSi(CH_3)_3 \qquad (IX)$$

wherein w is in the range from 70 to 1,350.

The present invention also provides a lubricating silicone film formed from the composition, the film comprising a crosslinked siloxane polymer or copolymer matrix having distributed therein a lubricating siloxane polymer. The film is capable of adhering to substrate surfaces and its lubricating surface is substantially dry to the touch.

Preferably, the molecular weight per crosslink is in the range from 5000 to 110,000 and/or the film comprises in the range from 3% to 35% of the siloxane polymer (1) and 3% to 50% of the siloxane polymer

(2).

The present invention also provides an article having a substrate surface, said surface having an adherent coating of at least a partially cured film formed from a composition of the invention, the film comprising a crosslinked siloxane polymer or copolymer matrix having distributed therein a lubricating siloxane polymer or copolymer.

Preferably the substrate surface is formed of a metallic or plastics material, and, when the surface is a metal surface, it preferably has a fine cutting edge.

The present invention also provides a hypodermic needle having a coating as defined above.

The needle is preferably one wherein the siloxane polymer (1) of the siloxane matrix comprises a mixture of two polymers of the formula VII having respective average molecular weights of approximately 16,000 and 38,000; the siloxane polymer (2) of the matrix has an average molecular weight of approximately 1900; and the non-reactive lubricating siloxane (b) has an average molecular weight of approximately 16,000; and/or wherein the weight ratio of reactive component (a) to non-reactive component (b) is in the range of 20:80 to 30:70; and/or wherein the weight ratio of the siloxane polymer (1) to the siloxane polymer (2) in the reactive mixture (a) is in the range of from 30:70 to 40:60.

The present invention provides a process for producing a lubricating siloxane film having a dry to the touch, non-tacky surface, the process comprising the steps of:

(i) providing a reactive component (a) comprising said siloxane polymers (1), (2) and (3);

(ii) mixing the mixture of step (i) with said non-reactive lubricating siloxane polymer; and

(iii) curing the resulting mixture.

The viscosity of the non-reactive polymer (a) and the weight ratio of the non-reactive component to the reactive component (b) are the two most significant variables influencing the properties of the final coatings and films. When applied to a hypodermic needle as a coating, the penetration, drag, retract and adhesion to needle-surface are affected by these variables. Generally, the lower viscosity compositions cure to better lubricating films and coatings. Additionally, better lubricating properties are also obtained in the resultant films and coatings if the weight ratio of the non-reactive lubricating component (b) to reactive component (a) is increased. Penetration forces are lowest when the ratio of the crosslinker (i.e. the siloxane polymer (2) of the reactive component (a)) to the chain extender (i.e. the siloxane polymer (3) of the reactive component (a)) is lowest. The weight ratio of the reactive component (a) to the non-reactive component (b) is preferably in the range from 20:80 to 30:70.

The compositions of the present invention are useful on a variety of materials and substrates such as metal and plastics and in applications where dry lubrication is required. The compositions have excellent adherent properties when cured and, if used in the proper thickness, may serve as relatively permanent lubricative films.

Curing the reactive portion can be accomplished by conventional methods well know in the art. For example, heat curing via oven or radio frequency (RF) are useful methods as well as the use of gamma radiation. Any mechanism which will initiate the hydrosilylation reaction is a useful curing technique. In the case of oven curing, temperatures should range from about 150° to about 180°C and residence time in the oven is generally about 30 to about 40 seconds, depending on the precise formulation. If RF techniques are used, the coil should conduct enough heat to obtain a substrate surface temperature of about 180° to about 240°C. At these temperatures, only about 2 to about 4 seconds are required for cure. This technique is particularly useful on hypodermic needles, catheters and cutting edges where production costs can be lowered significantly. If gamma radiation techniques are used, the need for hydrosilylation initiating catalyst is eliminated, since the radiation will start the cure. This technique has the advantage of sterilizing as well, which is useful in medical applications.

The inventive compositions can be partially cured to attach them to the substrate, and then fully cured at a later time. For example, air drying will permit partial cure. The compositions are initially fluid and can be applied directly to the substrate in any suitable manner, for example by dipping, brushing or spraying. The exact thickness of the coating does not appear to be critical and very thin coatings, e.g., one or two microns exhibit effective lubricating properties. While not necessary for operability, it is desirable that the thickness of the coating be substantially uniform throughout.

The inventive compositions can be applied from an inert, solvent carrier, such as non-toxic chlorinated or fluorinated hydrocarbons. For example, 1,1,2-trichloro-1,2,2-trifluoroethane, freon and the like are useful. Conventional hydrocarbon solvents such as alkanes, toluene, petroleum ether and the like are also useful in applications where toxiciology is not considered important.

The compositions when cured have two distinct properties which are related to the two distinct components. The reactive component gives the cured product its surface adherent properties, allowing the film to coat and stick to the substrate. Chemical attraction of the hydrogen functionality on the crosslinked

film to the oxides and hydroxyl groups on the substrate surface are believed to be primarily responsible for the adhesion, although some physical adhesion may also be occurring. The adhesion thus provides a definite advantage in that they do not wipe off and remain on the substrate without creep or migration over long periods of storage time. The films are dry to the touch and are less likely to trap dust and dirt as the prior art compositions.

As previously stated, the non-reactive component provides the lubricating property to the film. Lubrication is experienced on the non-adherent surface of the film, that is, on the exposed side. Without wishing to be bound to any one theory, the non-reactive polymer chains are believed to fit within the voids of the cured reactive component. Figure 1 provides an illustration of the probable chemical configuration of the cured film on a substrate. Chains labelled "A" represent the siloxanes of the reactive component having vinyl functionality. Those labelled "B" represent siloxanes of the reactive component having hydrogen functionality. The substrate is depicted as a metal surface where metal oxides (MO) and metal hydroxyl groups (MOH) are present. Chemical bonds can be seen between the functional groups of the reactive components and the oxide and hydroxyl groups of the substrate. The chains labelled "C" represent the non-reactive component, which can be seen to be physically entrapped within the voids of the reactive chains.

To prepare the inventive compositions, appropriate quantities of the three siloxanes required for the reactive component are mixed along with a catalyst solution. It is preferred that the vinyldimethylsilyl terminated PDMS polymers be mixed together first, followed by addition of the catalyst and finally the second and third siloxanes, e.g., the cross-linker and chain-extender. Mixing takes place for five to fifteen minutes at room temperature. This reactive portion is then combined with the non-reactive polymer and mixed for about five minutes at room temperature. The mixture is then diluted with a solvent, for example 1,1,2-trichloro-1,2,2-trifluoroethane to prepare a 4 weight % solids concentration. The fluid mixture is then ready to be applied to a substrate by one of the aforementioned techniques, and subsequently cured to a film.

The films and coatings of the instant invention may have an average molecular weight per crosslink of about 5,000 to about 110,000 and preferably about 15,000 to about 37,000.

The following examples serve to provide further appreciation of the invention but are not meant in any way to restrict the effective scope of the invention. All percentages are by weight of the total composition unless otherwise specified.

## EXAMPLE 1

This example is intended to show the criticality of process conditions and materials used in the preparation of the coating formulations. Six formulations were prepared according to the procedure below and needles were coated therefrom. All of the formulations in Table I were prepared according to the following procedure.

A homogenous solution of the vinyldimethylsilyl terminated polydimethylsiloxane polymers was prepared by mixing 1,000 centistoke and 10,000 centistoke polymers for 3-5 minutes. To this solution was added chloroplatinic acid catalyst in a sufficient amount to catalyze the silane to vinylsilane addition reaction, and the solution was mixed for about 3-5 minutes. A separate homogenous solution of the crosslinker and chain-extender was prepared at room temperature by mixing for 3-5 minutes. The vinyl functional polymer solution was added to the crosslinker/chain-extender solution (hydrogen functional solution) and mixed for 3-5 minutes. To this reactive polymer solution was added the non-reactive silicone polymer and the combined sample is mixed for 3-5 minutes at room temperature. Samples of the inventive coating compositions were diluted with 1,1,2-trichloro-1,2,2-trifluoroethane to a concentration of 4 wt.% solids.

TABLE I

COATING FORMULATIONS (weight %)

| Ingredients | A | B | C | D | E | F |
|---|---|---|---|---|---|---|
| **A. Reactive Component** | | | | | | |
| $ViMe_2$ Siloxane Polymer(1) | 4.5 | 10.8 | 8.4 | 21 | 1.2 | 14 |
| $ViMe_2$ Siloxane Polymer(2) | 10.5 | 7.2 | 3.6 | 9 | 1.8 | 21 |
| Crosslinker(3) | 1.75 | 1.5 | .9 | 1.5 | .875 | .75 |
| Chain-extender(4) | 33.25 | 10.5 | 17.1 | 18.5 | 6.125 | 14.25 |
| **B. Non-Reactive Component** | | | | | | |
| Weight % Lubricative Polymer/Viscosity(5) | 50/350 | 70/350 | 70/1000 | 50/12,500 | 90/12,500 | 50/110,000 |

(1) $ViMe_2$ Siloxane Polymer: 1,000 centistokes (cstks.)

```
         Me    Me     Me
         |     |      |
CH2=CH   SiO(SiO)xSi  CH=CH2
         |     |      |
         Me    Me     Me
```

(2) $ViMe_2$ Siloxane Polymer: 10,000 cstks.

```
        Me   Me    Me
CH2=CH  SiO(SiO)xSi  CH=CH2
        Me   Me    Me
```

(3) Crosslinker:

```
          Me
Me3 SiO(SiO)xSiMe3, 25 cstks
          H
```

(4) Chain-extender: 500 cstks.

```
 Me   Me    Me
HSiO(SiO)xSiH
 Me   Me    Me
```

(5) Lubricative Polymers:

```
        Me
Me3SiO(SiO)xSiMe3
        Me
```

EXAMPLE 2

This example is intended to demonstrate by comparative tests the advantages the inventive compositions have over the prior art. The tests were specifically designed to compare the lubrication properties of the inventive compositions against the prior art.

Each of the compositions in Table I was used as a coating material for clean stainless steel, 16 gauge hypodermic needles. Five needle samples were used for each composition. The needles coated with the inventive compositions (A-F) were mechanically dipped 19mm (3/4") deep and withdrawn at a rate of about 12mm/sec. The comparative compositions of the prior art (G-H) were similarly dipped and withdrawn at the rate of about 6mm/sec. Certain of the control needles were dipped 19mm (3/4") deep in 4 wt % solution of the commercial version of the composition described in the aforementioned U.S. Patent 3,574,673. Other control needles were dipped similarily in a 4 wt % solution of 12,500 centistoke Dow Corning 360 Fluid. The

control polymer solutions all used 1,1,2-trichloro-1,2,2-trifluoroethane as the solvent.

Coating of all the needles was done using a machine which controlled the rate of dipping and. withdrawal. The thickness of the coating is related to the speed of withdrawal of the substrate. In the case of needles, an optimum speed for obtaining a coating with the highest degree of lubricity was chosen through routine experimentation and experimental modeling. Generally, the faster the substrate is withdrawn from the fluid composition, the thicker the resultant coating. This would be expected since there would be less time for the fluid to run off the substrate.

Speeds of withdrawal for all formulations tested were chosen to maximize the degree of lubricity. The speeds for the control formulations were the conventional rates used for high speed assembly-line coating. The speeds for maximizing lubricity of the inventive compositions was determined to be about 12.7mm/sec; and was about 6.35mm/sec for the control compositions. Thus, the respective rates were previously determined by routine experimentation to be the appropriate ones for obtaining a coating with the highest degree of lubricity.

The coated needles were then tested for penetration, drag, retract and catheter/needle adhesion. A natural isoprene rubber, ASTM D-2000 type AA was used as a test membrane through which the needles were pierced. The force required for the 16 gauge needles to pierce a 1/16" X 1 X 1-1/8 membrane was recorded as the penetration value . The membrane was held by a clamp assembly at a 45° angle and the penetration and withdrawal was performed by a Model 1122 Instron Tensile machine. A fresh, unpierced membrane was used for each measurement.

For purposes of this invention, the drag force is defined as the force required to slide the needle surface through the punctured membrane when inserting the needle through the membrane. That is, it is the functional force between the needle and the membrane after the needle has punctured the membrane and is continued to be moved in relation to the membrane.

The retract force is the force required to slide the needle surface through the membrane when withdrawing the needle.

The catheter/needle adhesion force is the force required to separate the catheter from the needle at their point of adhesion.

Turning to Table II, it is clear that the inventive compositions A-F demonstrated significantly lower values for the force required to penetrate the skin, drag through the skin and for retraction, than the two commercially available silicone lubricants shown. The values for catheter/needle adhesion indicated acceptable lubricating properties such that the needle can be easily separated from the catheter when the needle is withdrawn from the catheter to needle assembly. It is apparent that the inventive compositions have excellent adherent properties on the substrate to which they are applied, yet are non-tacky and do not transfer to adjacent surfaces. Rather, these adjacent surfaces easily slide over the coated surface due to the lubricating properties of the inventive compositions. The inventive compositions may be employed as coating on a variety of materials and substrates, thereby imparting their lubricating effects. It is apparent that the inventive compositions exhibit improved lubricity over the prior art.

TABLE II

| Needles Coated with Coating: | Penetration | Drag | Retract | Catheter/Needle Adhesion |
|---|---|---|---|---|
| A | 242 | 37 | 37 | 120 |
| B | 257 | 44 | 41 | 95 |
| C | 246 | 18 | 22 | 57 |
| D | 270 | 45 | 44 | 158 |
| E | 288 | 20 | 41 | 70 |
| F | 262 | 56 | 56 | 97 |
| G* | 282 | 76 | 82 | 189 |
| H** | 297 | 29 | 48 | 114 |

* Comparative Composition from US Patent 3,574,073 (Dow Corning MDX-4-4159 commercial needle lubricant)

** Trimethylsilyl terminated PDMS (Dow Corning medical grade 360 fluid silicone)

The invention being thus described, it will be obvious that the same may be varied in many ways. Such variations are not to be regarded as a departure from the spirit and scope of the invention and all such

modifications are intended to be included within the scope of the claims.

Throughout the description and claims the term "pendent (or "pendant") when used in relation to a hydrogen atom is intended to mean a hydrogen atom that is bonded to a silicon atom which forms part of the main chain of a polymer, but which is not a terminal silicon atom. A "terminal hydrogen atom" is intended to mean any hydrogen atom which is bonded to a terminal silicon atom in a chain.

## Claims

**1.** A film-forming composition comprising:

(a) a reactive component for providing adhesion, said reactive component comprising:

(1) a siloxane polymer having a formula selected from:

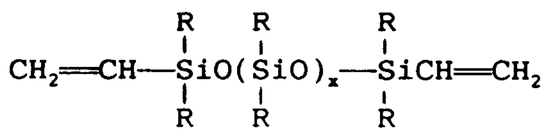

(I)

and

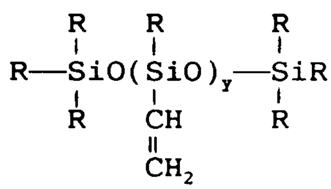

(II)

and

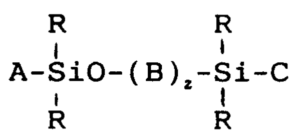

(III)

wherein R is selected from $C_{1-20}$ alkyl, haloalkyl, aryl, haloaryl, cycloalkyl, silacyclopentyl, aralkyl and mixtures thereof; x is from 60 to 1000; y is from 3 to 25; z is 3 to 25 to 60 to 1000; $(B)_z$ is a mixture or combination, in any order, of

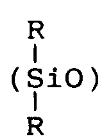

and

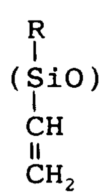

units such that the total number of units is equal to z, each of A and C is independently vinyl or R, wherein R is as defined above;

(2) a siloxane cross-linking polymer having the formula:

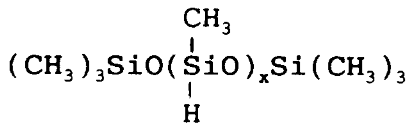 (IV)

wherein x is from 8 to 12; and

(3) a siloxane chain-extending polymer having the formula:

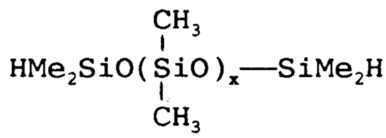 (V)

wherein x is from 140 to 160; and

(b) a non-reactive lubricating siloxane polymer component having the formula:

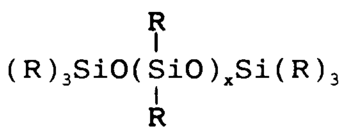 (VI)

wherein R is selected from $C_{1-20}$ alkyl, haloalkyl, aryl, haloaryl, cycloalkyl, silacyclopentyl, aralkyl, and mixtures thereof; and x is from 20 to 1,350;

wherein the mole ratio of vinyl groups to hydrogen atoms is within the range of from 0.010:1 to 0.20:1.

**2.** A composition according to claim 1, wherein the siloxane polymer (1) of the reactive component (a) is present in an amount of from 3% to 35% by weight of the total composition.

**3.** A composition according to claim 1 or 2, wherein the siloxane cross-linking polymer (2) of the reactive component (a) is present in an amount of from 0.3% to 5.5% by weight of the total composition.

**4.** A composition according to any of claims 1 to 3, wherein the siloxane chain-extending polymer (3) of the reactive component (a) is present in an amount of from 2.5% to 50.0% by weight of the total composition.

**5.** A composition according to any of claims 1 to 4, wherein the mole ratio of hydrogen atoms of the cross-linking polymer (2) to the hydrogen atoms of the chain-extending polymer (3) is from 5.0:1 to 20:1.

**6.** A composition according to any of claims 1 to 5, wherein the viscosity of the reactive component (a) from 100 to 100,000 centistokes.

**7.** A composition according to any of claims 1 to 6, wherein the average molecular weight per cross-link of the total reactive component (a) is from 5,000 to 75,000.

**8.** A composition according to any of claims 1 to 7, wherein the non-reactive lubricating component (b) is present in an amount of from 10% to 90% by weight of the total composition.

**9.** A composition according to any of claims 1 to 8, wherein the viscosity of the non-reactive siloxane polymer component (b) is from 20 to 300,000 centistokes at room temperature.

**10.** A composition according to any of claims 1 to 9, wherein the average molecular weight of the non-reactive siloxane polymer component (b) is from 1900 to 100,000.

**11.** A composition according to any of claims 1 to 10, wherein there is additionally present a solution of a metallic catalyst.

**12.** A composition according to any of claims 1 to 11, wherein the siloxane polymer (1) having the formula (I) has a molecular weight of from 5,000 to 25,000; wherein the siloxane polymer (1) having the formula (II) has a molecular weight of from 30,000 to 75,000; and wherein the non-reactive lubricating siloxane polymer component (b) has a molecular weight of from 5,000 to 100,000.

**13.** A film formed from a composition according to any of claims 1 to 12.

**14.** An article having, on a surface thereof, a film according to claim 13,

**15.** An article according to claim 14, wherein the said surface is formed of a metallic or plastics material.

**16.** An article according to claim 15, wherein the said surface is formed of a metallic material and has a fine cutting edge.

**17.** An article according to claim 14 or 15, being a hypodermic needle.

**18.** A process for producing a film-forming composition, which comprises the steps of:
(i) admixing, in a first admixing step, a siloxane polymer (1) as defined in claim 1, a siloxane cross-linking polymer (2) as defined in claim 1, and a siloxane chain-extending polymer (3) as defined in claim 1; and
(ii) admixing, in a second admixing step, the admixture obtained by said first admixing step with a non-reactive lubricating siloxane polymer component (b) as defined in claim 1;
wherein the mole ratio of vinyl groups to hydrogen atoms is within the range of from 0.010:1 to 0.20:1.

**19.** A process for producing a film, which comprises the steps of:
(i) producing a film-forming composition by a process according to claim 18; and
(ii) curing the composition to form a film.

**Revendications**

**1.** Composition pour constituer un film comprenant :
(a) un composant réactif pour assurer l'adhésion, ledit composant réactif comprenant :
(1) un polymère siloxane ayant une formule choisie parmi :

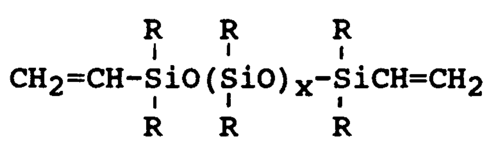

(I)

et

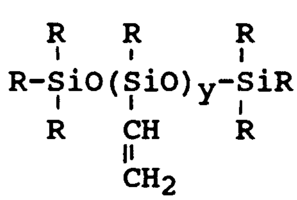

(II)

et

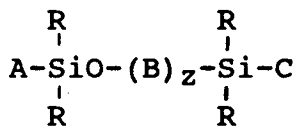 (III)

où R est choisi parmi les alkyles $C_{1-20}$, les haloalkyles, les aryles, les haloaryles, les cycloalkyles, le silacyclopentyle, les aralkyles et leurs mélanges; x va de 60 à 1000; y va de 3 à 25; z va de 3 à 25 à 60 à 1000; $(B)_z$ est un mélange ou une combinaison, dans n'importe quel ordre, d'unités

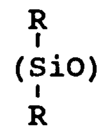

et

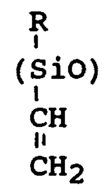

tel que le nombre total d'unités soit égal à z, chacun des A et C est indépendamment un vinyle ou R, où R est comme défini ci-dessus;
(2) un polymère siloxane de réticulation ayant la formule :

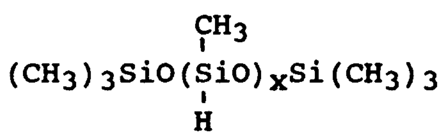 (IV)

où x va de 8 à 12; et
(3) un polymère siloxane d'allongement de chaîne ayant la formule :

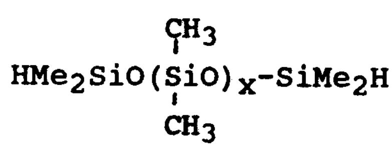 (V)

où x va de 140 à 160; et
(b) un polymère siloxane lubrifiant non réactif ayant la formule :

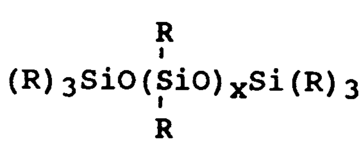 (VI)

où R est choisi parmi les alkyles $C_{1-20}$, les haloalkyles, les aryles, les haloaryles, les cycloalkyles, le silacyclopentyle, les aralkyles et leurs mélanges; et x va de 20 à 1350;

où le rapport molaire des groupes vinyle sur les atomes hydrogène est dans la gamme de 0,010:1 à 0,20:1.

**2.** Composition selon la revendication 1, où le polymère siloxane (1) du composant réactif (a) est présent en une quantité de 3% à 35% en poids de la composition totale.

**3.** Composition selon la revendication 1 ou 2, où le polymère siloxane de réticulation (2) du composant réactif (a) est présent en une quantité de 0,3% à 5,5% en poids de la composition totale.

**4.** Composition selon l'une quelconque des revendications 1 à 3, où le polymère siloxane d'allongement de chaîne (3) du composant réactif (a) est présent en une quantité dans la gamme de 2,5% à 50,0% en poids de la composition totale.

**5.** Composition selon l'une quelconque des revendications 1 à 4, où le rapport molaire des atomes d'hydrogène du polymère de réticulation (2) sur les atomes d'hydrogène du polymère d'allongement de chaîne (3) est de 5,0:1 à 20:1.

**6.** Composition selon l'une quelconque des revendications 1 à 5, où la viscosité du composant réactif (a) est de 100 à 100000 centistokes.

**7.** Composition selon l'une quelconque des revendications 1 à 6, où le poids moléculaire moyen par réticulation du composant réactif total (a) est de 5000 à 75000.

**8.** Composition selon l'une quelconque des revendications 1 à 7, où le composant lubrifiant non réactif (b) est présent en une quantité de 10% à 90% en poids de la composition totale.

**9.** Composition selon l'une quelconque des revendications 1 à 8, où la viscosité du composant polymère siloxane non réactif (b) est de 20 à 300000 centistokes à la température ambiante.

**10.** Composition selon l'une quelconque des revendication 1 à 9, où le poids moléculaire moyen du composant polymère siloxane non réactif (b) est de 1900 à 100000.

**11.** Composition selon l'une quelconque des revendications 1 à 10, contenant en outre une solution de catalyseur métallique.

**12.** Composition selon l'une quelconque des revendications 1 à 11, où le polymère siloxane (1) ayant la formule (I) a un poids moléculaire de 5000 à 25000; où le polymère siloxane (1) ayant la formule (II) a un poids moléculaire de 30000 à 75000; et où le composant polymère siloxane lubrifiant non réactif (b) a un poids moléculaire de 5000 à 100000.

**13.** Film formé avec une composition selon l'une quelconque des revendications 1 à 12.

**14.** Article ayant sur une surface un film selon la revendication 13.

**15.** Article selon la revendication 14, où ladite surface est constituée d'un matériau métallique ou plastique.

**16.** Article selon la revendication 15, où ladite surface est formée d'un matériau métallique et possède un mince bord coupant.

**17.** Article selon la revendication 14 ou 15, qui est une aiguille hypodermique.

**18.** Procédé pour produire une composition pour constituer un film comprenant les étapes consistant à :
(i) mélanger, dans une première étape de mélange, un polymère siloxane (1) comme défini dans la revendication 1, un polymère siloxane de réticulation (2) comme défini dans la revendication 1 et un polymère siloxane d'allongement de chaîne (3) comme défini dans la revendication 1; et
(ii) mélanger, dans une seconde étape de mélange, le mélange obtenu dans la dite première étape de mélange avec un composant polymère siloxane lubrifiant non réactif (b) comme défini dans la revendication 1;

où le rapport molaire des groupes vinyle sur les atomes d'hydrogène est dans la gamme de 0,010:1 à 0,20:1.

19. Procédé pour constituer un film, qui comprend les étapes consistant à :
(i) produire, une composition pour constituer un film par le procédé selon la revendication 18; et
(ii) durcir la composition pour constituer un film.

**Patentansprüche**

1. Eine filmbildende Mischung die enthält:
(a) eine reaktive, haftungvermittelnde Komponente welche umfasst:
(1) ein Siloxan-Polymer das eine der folgenden Formeln hat:

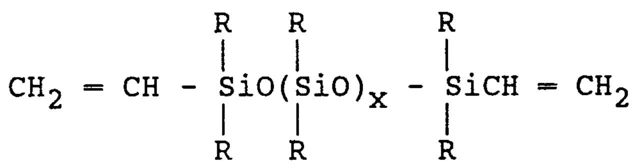
(I)

und

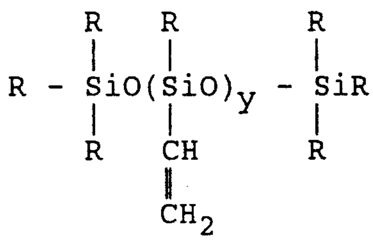
(II)

und

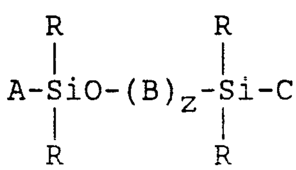
(III)

worin R aus $C_{1-20}$ ALkyl, Halogenalkyl, Aryl, Halogenaryl, Cycloalkyl, Siliziumcyclopentyl, Aralkyl und Mischungen davon ausgewählt ist; x geht von 60 bis 1000, y geht von 3 bis 25; z geht von 3 bis 25 bis 60 bis 1000; $(B)_z$ ist eine Mischung oder Kombination, in beliebiger Reihenfolge von

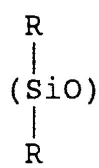 und 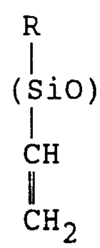 Einheiten,

so dass die totale Anzahl der Einheiten gleich z ist, jede von A und C ist unabhängig Vinyl oder

R, wobei R oben definiert ist;
(2) ein Siloxan-Cross-link-Polymer mit der Formel:

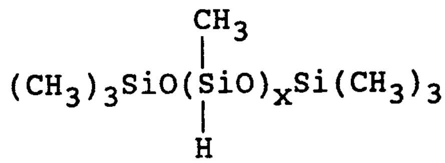

(IV)

worin x von 8 bis 12 geht; und
(3) eine siloxan-kettenbildendes Polymer mit der Formel:

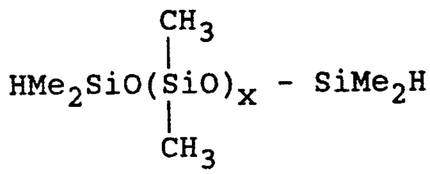

(V)

worin x von 140 bis 160 geht; und
(b) eine nichtreaktive, schmierende Siloxan-Polymer-Komponente mit der Formel:

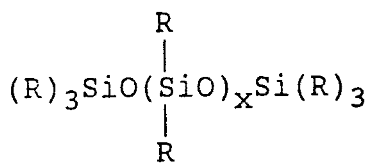

(VI)

worin R aus $C_{1-20}$ Alkyl, Halogenalkyl, Aryl, Halogenaryl, Cycloalkyl, Cycloalkyl, Siliziumzyklopentyl, Aralkyl, oder Mischungen davon ausgewählt ist; und x geht von 20 bis 1'350;
worin das Molverhältnis von Vinylgruppen zu Wasserstoffatomen im Bereich von 0.010:1 bis 0.20:1 liegt.

**2.** Eine Mischung nach Anspruch 1, in der das Siloxan-Polymer (1) der reaktiven Komponente (a) 3% bis 35% des Gesamtgewichts der gesamten Mischung beträgt.

**3.** Eine Mischung nach Anspruch 1 oder 2, in der das Siloxan-Cross-link-Polymer der reaktiven Komponenten 0.3% bis 5.5% des Gesamtgewichts der gesamten Mischung beträgt.

**4.** Eine Mischung nach einem der Ansprüche 1 bis 3, in der das siloxan-kettenbildende Polymer (3) der reaktiven Komponente (a) 2.5% bis 50% des Gesamtgewichts der gesamten Mischung beträgt.

**5.** Eine Mischung nach einem der Ansprüche 1 bis 4, in der das Molverhältnis von Wasserstoffatomen des Cross-link-Polymers (2) zu den Wasserstoffatomen des kettenbildenden Polymers (3) im Bereich von 5.0:1 bis 20:1 liegt.

**6.** Eine Mischung nach einem der Ansprüche 1 bis 5, bei der die Viskosität der reaktiven Komponente (a) im Bereich von 100 bis 100'000 Centistokes liegt.

**7.** Eine Mischung nach einem der Ansprüche 1 bis 6, bei der das mittlere Molekulargewicht pro Cross-link der gesamten reaktiven Komponente (a) zwischen 5'000 und 75'000 liegt.

**8.** Eine Mischung nach einem der Ansprüche 1 bis 7, bei der die nichtreaktive, schmierende Komponente (b) 10% bis 90% des Gesamtgewichts der gesamten Mischung beträgt.

**9.** Eine Mischung nach einem der Ansprüche 1 bis 8, bei der die Viskosität der nichtreaktiven Siloxan-Polymer-Komponente (b), bei Zimmertemperatur, zwischen 20 und 300'000 Centistokes liegt.

**10.** Ein Mischung nach einem der Ansprüche 1 bis 9, bei der das mittlere Molekulargewicht der nichtreaktiven Siloxan-Polymer-Komponente (b) im Bereich von 1900 bis 100'000 liegt.

**11.** Eine Mischung nach einem der Ansprüche 1 bis 10, welche zusätzlich eine Lösung eines metallischen Katalysators enthält.

**12.** Eine Mischung nach einem der Ansprüche 1 bis 11, in der das Siloxan-Polymer (1), welches die Formel (I) aufweist ein Molekulargewicht das im Bereich von 5'000 bis 25'000 liegt; worin das Siloxan-Polymer (1), das die Formel (II) aufweist, ein Molekulargewicht im Bereich von 30'000 bis 75'000 hat; und die nichtreaktive, schmierende Siloxan-Polymer-Komponente (b) ein Molekulargewicht im Bereich von 5'000 bis 100'000 hat.

**13.** Ein Schicht, die aus einer Mischung nach einem der Ansprüche 1 bis 12 gebildet ist.

**14.** Ein Produkt, das auf einer Oberfläche eine Schicht nach Anspruch 13 hat.

**15.** Ein Produkt nach Anspruch 14, bei welchem die Oberfläche aus metallischem oder Plastik-Material besteht.

**16.** Ein Produkt nach Anspruch 15, bei welchem die genannte Oberfläche aus einem metallischem Material eine scharfe Schneidkante aufweist.

**17.** Ein Produkt nach Anspruch 14 oder 15, welches eine hypodermale Nadel ist.

**18.** Ein Verfahren zum Herstellen einer schichtbildenden Mischung, welches die folgenden Schritte enthält:
(i) in einem ersten Schritt vermischen eines Siloxan-Polymers (1) gemäss Anspruch 1, eines Siloxan-Cross-link-Polymers (2) gemäss Anspruch 1 und eines siloxan-kettenbildendes Polymers (3) gemäss Anspruch 1; und
(ii) in einem zweiten Schritt, vermischen der Mischung des ersten Schritts mit einem nichtreaktiven, schmierenden Siloxan-Polymer-Komponente (b) gemäss Anspruch 1;
wobei das Molverhältnis der Vinyl-Gruppen zu den Wasserstoffatomen im Bereich von 0.010:1 bis 0.20:1 liegt.

**19.** Ein Verfahren zum Herstellen einer Schicht, welches die folgenden Schritte enthält:
(i) Herstellen einer schichtbildenden Mischung nach Anspruch 18; und
(ii) aushärten der Mischung, sodass eine Schicht gebildet wird.

FIG. 1

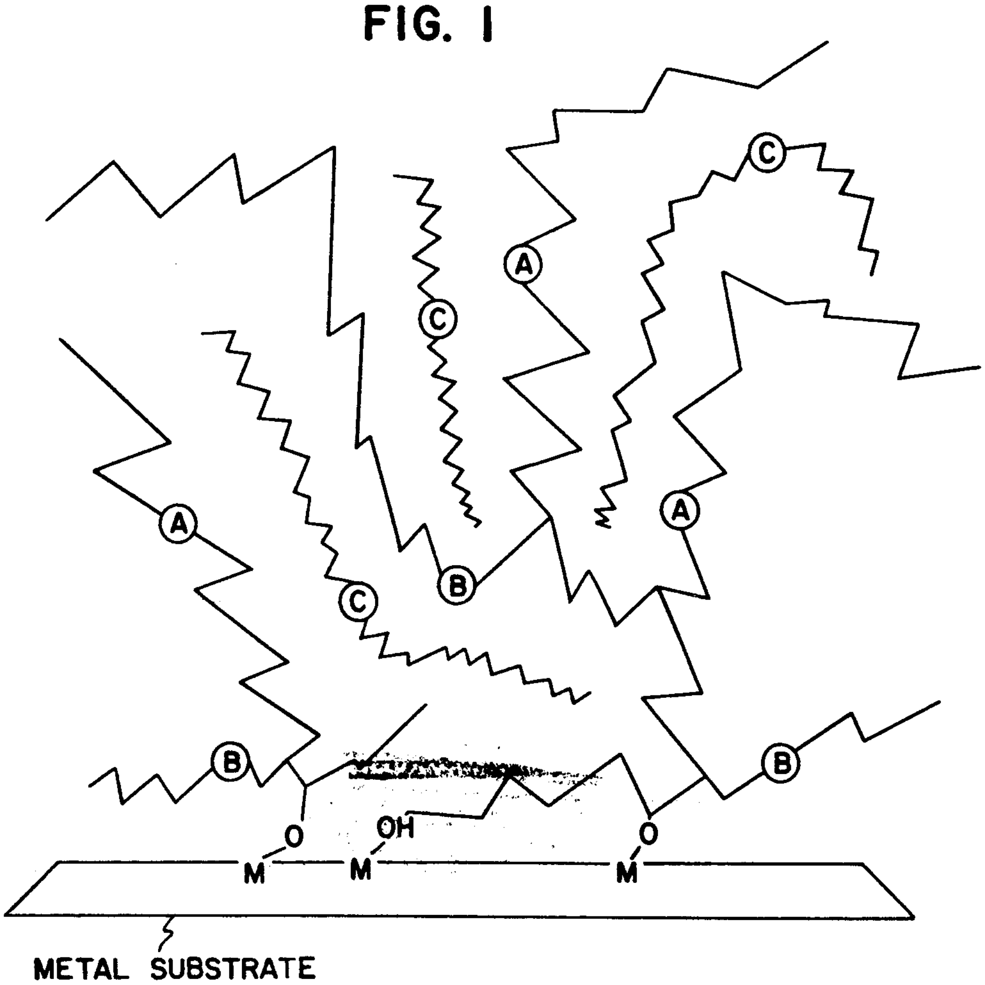


Ⓐ VINYL FUNCTIONALITY OF REACTIVE COMPONENT
Ⓑ HYDROGEN FUNCTIONALITY OF REACTIVE COMPONENT
Ⓒ LUBRICATING POLYMER
MO- METAL OXIDES
MOH- METAL HYDROXYL GROUPS